# EUROPÄISCHE PATENTSCHRIFT

(11) **EP 0 689 415 B1**
(45) Veröffentlichungstag und Bekanntmachung des Hinweises auf die Patenterteilung: **16.06.1999**
(21) Anmeldenummer: 94909108.6
(22) Anmeldetag: 03.03.1994
(51) Int. Cl.: A61K 7/00, A61K 7/50, A61K 7/48

(54) **NIEDRIGVISKOSE KOSMETISCHE UND PHARMAZEUTISCHE EMULSIONEN**
LOW-VISCOSITY COSMETIC AND PHARMACEUTICAL EMULSIONS
EMULSIONS COSMETIQUES ET PHARMACEUTIQUES DE BASSE VISCOSITE

(30) Priorität: 23.03.1993 DE 4309390
(43) Veröffentlichungstag der Anmeldung: 03.01.1996
(73) Patentinhaber: Beiersdorf Aktiengesellschaft, 20245 Hamburg (DE); Solvay Fluor und Derivate GmbH, D-30173 Hannover (DE)
(72) Erfinder: LEMM, Christel, D-21614 Buxtehude (DE); PAPE, Wolfgang, D-22041 Hamburg (DE); SCHNEIDER, Günther, D-20251 Hamburg (DE); TIMM, Stephanie, D-25421 Pinneberg (DE); SIEMANOWSKI, Werner, D-47495 Rheinberg (DE); UHLIG, Karl-Heinz, D-47802 Krefeld (DE)
(86) Internationale Anmeldenummer: EP9400622
(87) Internationale Veröffentlichungsnummer: WO9421215

(56) Entgegenhaltungen:
- EP-A- 0 379 658
- EP-A- 0 451 461
- WO-A-92/18227
- GB-A- 2 140 297
- CHEMICAL ABSTRACTS, vol. 108, no. 14, 4. April 1988, Columbus, Ohio, US; abstract no. 114726, M. OCHIAI 'Oil-in-water emulsions with glycerol ester surfactants' & JP,A,62 266 135 (POLA CHEMICAL INDUSTRIES) 18. November 1987

## Beschreibung

Die vorliegende Erfindung betrifft niedrigviskose stabile Emulsionen, insbesondere O/W-Emulsionen, Verfahren zu ihrer Herstellung, ihre Verwendung für kosmetische und medizinische Zwecke sowie die Verwendung bestimmter Emulgatoren zur Regulierung der Viskosität von Emulsionen, insbesondere von O/W-Emulsionen.

Emulsionen des Standes der Technik haben den Nachteil, daß sie meistens hochviskos sind, daß ihre Viskosität in nicht befriedigendem Maße regulierbar ist, oder daß sie zwar niedrigviskos aber instabil sind. Instabil bedeutet hier, daß solche Emulsionen sich leicht in die Öl- und Wasserphase trennen.

Zwar bedeutet hohe Viskosität nicht automatisch, daß die entsprechenden Emulsionen aus kosmetischen oder pharmazeutischen Erwägungen inakzeptabel wären. Dennoch erscheint in vielen Fällen eine niedrigviskose Emulsion kosmetisch eleganter als einer höherviskose.

Aus EP-A1-0 345 586 ist ein Verfahren zur Herstellung niedrigviskoser Öl-in-Wasser-Emulsionen (O/W-Emulsionen) von Ölkomponenten bekannt, wobei diese Ölkomponenten aus bestimmten Fettsäuremono- und/oder -diestern und gegebenenfalls Fettsäuretriglyceriden bestehen.

Das am angegebenen Orte vorgeschlagene Verfahren ist jedoch kompliziert und nicht in allen Fällen praktikabel.

Aufgabe der vorliegenden Erfindung war daher, den Nachteilen des Standes der Technik Abhilfe zu schaffen.

Es wurde überraschend gefunden, und darin liegt die Lösung all dieser Aufgaben, daß
Verfahren zur Regulation der Viskosität von Emulsionen, dadurch gekennzeichnet, daß man Emulsionen, welche als Emulgator oder Emulgatoren einen oder mehrere nichtionische Emulgatoren, insbesondere eine oder mehrere Alkancarbonsäuren und/oder deren Alkalisalze enthalten, einen oder mehrere Monocarbonsäureester des Di- und/oder Triglycerins bis zum Erreichen der gewünschten Viskosität zusetzt, sowie
die Verwendung eines oder mehrerer Monocarbonsäureester des Di- und/oder Triglycerins zur Regulation der Viskosität von Emulsionen, welche als Emulgator oder Emulgatoren einen oder mehrere nichtionische Emulgatoren, insbesondere eine oder mehrere Alkancarbonsäuren und/oder deren Alkalisalze enthalten, sowie
Verfahren zur Regulation der Viskosität von Emulsionen, dadurch gekennzeichnet, daß man Emulsionen, welche als Emulgator oder Emulgatoren einen oder mehrere Monocarbonsäureester des Di- und/oder Triglycerins enthalten, einen oder mehrere nichtionische Emulgatoren, insbesondere eine oder mehrere Alkancarbonsäuren und/oder deren Alkalisalzen bis zum Erreichen der gewünschten Viskosität zusetzt, sowie
die Verwendung eines oder mehrerer nichtionischer Emulgatoren, insbesondere einer oder mehrerer Alkancarbonsäuren und/oder deren Alkalisalzen zur Regulation der Viskosität von Emulsionen, welche als Emulgator oder Emulgatoren einen oder mehrere Monocarbonsäureester des Di- und/oder Triglycerins enthalten,
den Nachteilen des Standes der Technik abhelfen.

Insbesondere vorteilhaft im Sinne der vorliegende Erfindung werden Emulgatorengemische aus
(1) einem oder mehreren nichtionischen Emulgatoren, gewählt aus der Gruppe der Glycerinfettsäureester, Polyglycerinfettsäureester, Sorbitanfettsäureester, Glucosefettsäurester und Saccharosefettsäureester, wobei die diesen Estern zugrundeliegenden Fettsäuren verzweigte und/oder unverzweigte Alkancarbonsäuren mit 10 - 20 C-Atomen darstellen, sowie aus der Gruppe der Alkancarbonsäuren selbst bzw. deren Alkalisalzen und
(2) einem oder mehreren Monocarbonsäureestern des Di- und/oder Triglycerins, verwendet.

Zwar werden in den Schriften EP-A-0 451 461, GB-A-2 140 297 und Chemical Abstracts, Vol. 108, Nr. 114726 bestimmte Vertreter der erfindungsgemäß vorteilhaften Einsatzstoffe bzw. Emulsionen mit einem Gehalt an solchen Einsatzstoffen geschildert. Dennoch konnten diese Schriften nicht den weg zur vorliegenden Erfindung ebnen.

Erfindungsgemäß müssen die nichtionischen Emulgatoren O/W-Emulgatoren darstellen und sollen nichtethoxyliert sein.

Besonders wird als nichtionischer Emulgator Sorbitanmonostearat verwendet.

Erfindungsgemäß zeichnen sich die Alkancarbonsäuren und/oder deren Alkalisalze vorteilhaft durch die Struktur 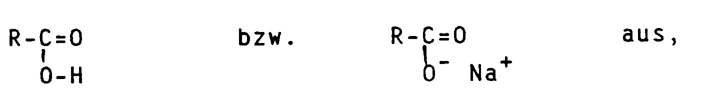 wobei R einen unverzweigten Alkylrest mit 13 - 21 C-Atomen darstellt.

Bevorzugt stellt R einen unverzweigten Alkylrest mit 15 oder 17 C-Atomen dar, also Palmitin- oder Stearinsäure bzw. Natriumpalmitat oder Natriumstearat. Es ist vorteilhaft, Gemische aus Palmitin- und Stearinsäure bzw. Natriumpalmitat und Natriumstearat zu verwenden.

Die erfindungsgemäßen Alkalisalze werden den entsprechenden Alkancarbonsäuren bevorzugt.

Erfindungsgemäß liegen die Di- bzw. Triglycerineinheiten der erfindungsgemäßen Monocarbonsäureester als lineare, unverzweigte Moleküle, also über die jeweiligen OH-Gruppen in 1- bzw. 3-Stellung veretherte "Monoglycerinmoleküle" vor.

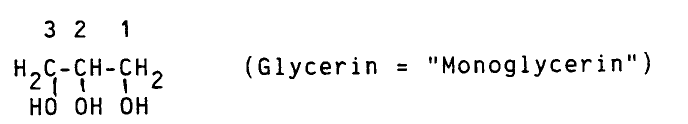

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäureester des Diglycerins sind vorzugsweise Monocarbonsäuremonoester und bevorzugt durch folgende Struktur gekennzeichnet (Substitutionspositionen angegeben): 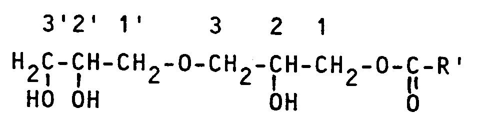 wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind vorzugsweise Monocarbonsäuremonoester und bevorzugt durch folgende Struktur gekennzeichnet (Substitutionspositionen angegeben): 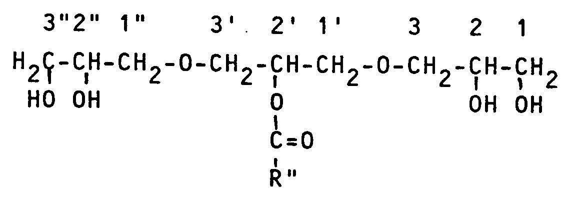 wobei R'' einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die

| | |
|---|---|
| Hexansäure (Capronsäure) | (R' bzw. R'' = -C₅H₁₁), |
| Heptansäure (Önanthsäure) | (R' bzw. R'' = -C₆H₁₃), |
| Octansäure (Caprylsäure) | (R' bzw. R'' = -C₇H₁₅), |
| Nonansäure (Pelargonsäure) | (R' bzw. R'' = -C₈H₁₇), |
| Decansäure (Caprinsäure) | (R' bzw. R'' = -C₉H₁₉), |
| Undecansäure | (R' bzw. R'' = -C₁₀H₂₁), |
| 10-Undecensäure (Undecylensäure) | (R' bzw. R'' = -C₁₀H₁₉), |
| Dodecansäure (Laurinsäure) | (R' bzw. R'' = -C₁₁H₂₃), |
| Tridecansäure | (R' bzw. R'' = -C₁₂H₂₅), |
| Tetradecansäure (Myristinsäure) | (R' bzw. R'' = -C₁₃H₂₇), |
| Pentadecansäure | (R' bzw. R'' = -C₁₄H₂₉), |
| Hexadecansäure (Palmitinsäure) | (R' bzw. R'' = -C₁₅H₃₁), |
| Heptadecansäure (Margarinsäure) | (R' bzw. R'' = -C₁₆H₃₃), |
| Octadecansäure (Stearinsäure) | (R' bzw. R'' = -C₁₇H₃₅). |

Besonders günstig werden R' und R'' gewählt aus der Gruppe der unverzweigten Alkylreste mit ungeraden C-Zahlen, insbesondere mit 9, 11 und 13 C-Atomen.

Im allgemeinen sind die Ester des Diglycerins denen des Triglycerins bevorzugt.

Ganz besonders günstig sind

| | |
|---|---|
| Diglycerinmonocaprinat (DMC) | R' = 9 |
| Triglycerinmonolaurat (TML) | R'' = 11 |
| Diglycerinmonolaurat (DML) | R' = 11 |
| Triglycerinmonomyristat (TMM) | R'' = 13. |

Als bevorzugter erfindungsgemäßer Monocarbonsäureester hat sich das Diglycerinmonocaprinat (DMC) erwiesen.

Die erfindungsgemäßen Monofettsäureester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß gleichermaßen vorteilhaft sind die 2S2'S-, die 2R2'S-, die 2S2'R- und die 2R2'R-Konfiguration.

Bei den Triglycerinestern sind die 2-, die 2' und die 2''-Position Asymmetriezentren. Erfindungsgemäß gleichermaßen vorteilhaft sind die 2S2'S2''S-, die 2R2'S2''S-, die 2S2'R2''S-, die 2R2'R2''S-, die 2S2'S2''R, die 2R2'S2''R-, die 2S2'R2''R- und die 2R2'R2''R-Konfiguration.

Es hat sich als günstig herausgestellt, racemische Gemische der Stereoisomeren zu verwenden.

Nach einer vorteilhaften Ausführungsform der vorliegenden Erfindung werden Gemische eines oder mehrerer Monocarbonsäureester des Diglycerins mit einem oder mehreren Monocarbonsäureestern des Triglycerins verwendet.

Nach einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit anderen in Kosmetika oder Pharmaka üblichen Wirkstoffen (Ersatzwirkstoffe), Hilfs-, Verschnitt- und/oder Zusatzstoffen eingesetzt.

Vorteilhaft liegen dann die Verschnittstoffe und/oder Ersatzwirkstoffe in einer Konzentration bis zu 50 Gew.-Teilen vor, bevorzugt bis zu 35 Gew.-Teilen, bezogen auf das 100 Gew.-Teile der Gesamtmenge, welche sich aus dem Monocarbonsäureester bzw. den Monocarbonsäureestern des Diglycerins und/oder des Triglycerins und diesen Ersatzwirkstoffen und/oder Verschnittstoffen zusammensetzt.

Nach noch einer weiteren vorteilhaften Ausführungsform der vorliegenden Erfindung werden ein oder mehrere Monocarbonsäureester des Diglycerins und/oder ein oder mehrere Monocarbonsäureester des Triglycerins in Kombination mit Monocarbonsäureestern des Glycerins (also des "Monoglycerins") eingesetzt. Dabei übernehmen diese Monocarbonsäureester des Glycerins die Rolle der Verschnittstoffe und/oder Ersatzwirkstoffe und werden vorzugsweise in einer Konzentration bis zu 50 Gew.-Teilen, bevorzugt bis zu 35 Gew.-Teilen eingesetzt, bezogen auf 100 Gew.-Teile der Gesamtmenge, welche sich aus dem Monocarbonsäureester bzw. den Monocarbonsäureestern des Diglycerins und/oder des Triglycerins und diesen Monocarbonsäureestern des Glycerins zusammensetzt.

Solche Monocarbonsäureester des Glycerins sind günstig gekennzeichnet durch eine Struktur wie folgt: 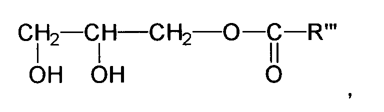 wobei R''' einen Kohlenwasserstoffrest, vorteilhaft einen verzweigten oder unverzweigten Alkyl- oder Alkenylrest von 5 bis 17 C-Atomen darstellt.

Die erfindungsgemäß erzielten Emulsionen sind besonders vorteilhaft dadurch gekennzeichnet, daß die Alkancarbonsäure oder die Alkancarbonsäuren oder das Alkalisalz oder die Alkalisalze einer oder mehrerer Alkancarbonsäuren in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zubereitung, vorliegt oder vorliegen.

Die erfindungsgemäß erzielten Emulsionen sind besonders vorteilhaft dadurch gekennzeichnet, daß der oder die Monocarbonsäureester des Di- und/oder Triglycerins in Konzentrationen von 0,01 - 10,00 Gew.-%, bevorzugt 0,05 - 5,00 Gew.-%, besonders bevorzugt 0,1 - 3,00 Gew.-%, jeweils bezogen auf das Gesamtgewicht der Zubereitung, vorliegt oder vorliegen.

Die erfindungsgemäßen Emulsionen können in Form von W/O- oder O/W-Emulsionen, z.B. Crèmes oder Lotionen vorliegen. Besonders bevorzugt sind O/W-Emulsionen.

Als übliche kosmetische oder pharmazeutische Trägerstoffe zur Herstellung der erfindungsgemäßen Emulsionen können neben Wasser, Fette, Öle, beispielsweise mineralische, pflanzliche und/oder Tierische Öle, ferner wachse, beispielsweise Bienenwachs, pflanzliche und mineralische Wachse, und Paraffine enthalten, darüberhinaus können Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als weitere Emulgatoren zur Herstellung der erfindungsgemäßen Emulsionen, und die in den Zubereitungen in geringer Menge, z.B. 0,10 bis 5,00 Gewichts.-%, bezogen auf die Gesamt-Zubereitung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen Emulsionen Parfüm, Farbstoffe, Antioxidantien (z.B. α-Tocopherol und seine Derivate oder Butylhydroxytoluol (BHT = 2,6-Di-Tert.-butyl-4-methylphenol) in Mengen von 0,01 bis 0,03 %, bezogen auf die Gesamtzubereitung), Suspendiermittel, Puffergemische oder andere übliche kosmetische Grundstoffe beigemischt werden.

Weitere fakultative Bestandteile der erfindungsgemäßen Emulsionen können Konservierungsmittel sein.

Vorteilhaft wird der pH-Wert der erfindungsgemäßen Formulierungen im schwach sauren bis schwach alkalischen Bereich eingestellt, bevorzugt von 4,0 - 9,0 , besonders bevorzugt von 5,5 - 8,5, insbesondere von 7,0 - 8,0.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Die Herstellung der kosmetischen oder pharmazeutischen Zubereitungen erfolgt in üblicher Weise, meist durch einfaches Vermischen unter Rühren, gegebenenfalls unter leichter Erwärmung. Die Fettphase und die Wasserphase werden vorteilhaft separat, gegebenenfalls unter Erwärmen hergestellt und dann emulgiert.

Ansonsten sind die üblichen Maßregeln für die Herstellung kosmetischer Formulierungen zu beachten, die dem Fachmann geläufig sind.

Es folgen vorteilhafte Ausführungsbeispiele der vorliegenden Erfindung.

| Beispiel 1 | |
|---|---|
| | Gew.-% |
| Sorbitanstearat | 2,50 |
| Vaseline | 2,50 |
| Paraffinöl DAB 9 | 14,00 |
| Hydrierte Kokosfettsäureglyceride | 1,00 |
| Triglycerinmonomyristat | 0,20 |
| Carbomer 934 | 0,20 |
| Cetylphosphat | 0,15 |
| 1,3-Butylenglycol | 2,00 |
| Parfum, Konservierungsmittel, Farbstoffe, Antioxidantien | q.s. |
| Wasser VES | ad 100,00 |

| Beispiel 2 | |
|---|---|
| | Gew.-% |
| Glycerylmonostearat | 0,50 |
| Stearinsäure | 2,50 |
| Vaseline | 3,00 |
| Paraffinöl DAB 9 | 15,00 |
| Diglycerinmonocaprinat | 0,50 |
| Carbomer 934 | 0,20 |
| Cetylphosphat | 0,15 |
| Glycerin | 3,00 |
| Dimethicone | 0,50 |
| Parfum, Konservierungsmittel, Farbstoffe, Antioxidantien | q.s. |
| Wasser VES | ad 100,00 |

| Beispiel 3 | |
|---|---|
| | Gew.-% |
| Glycerylmonostearat | 1,00 |
| Stearinsäure | 2,00 |
| Paraffinöl DAB 9 | 13,00 |
| Hydrierte Kokosfettsäureglyceride | 3,00 |
| Diglycerinmonocaprinat | 0,50 |
| Carbomer 934 | 0,20 |
| Cetylphosphat | 0,10 |
| Glycerin | 3,00 |
| Parfum, Konservierungsmittel, Farbstoffe, Antioxidantien | q.s. |
| Wasser VES | ad 100,00 |

| Beispiel 4 | |
|---|---|
| | Gew.-% |
| Sorbitanstearat | 2,50 |
| Vaseline | 2,00 |
| Paraffinöl DAB 9 | 13,00 |
| Hydrierte Kokosfettsäureglyceride | 1,50 |
| Triglycerinmonolaurat | 0,20 |
| Carbomer 934 | 0,20 |
| Cetylphosphat | 0,10 |
| Glycerin | 3,00 |
| Dimethicone | 0,30 |
| Parfum, Konservierungsmittel, Farbstoffe, Antioxidantien | q.s. |
| Wasser VES | ad 100,00 |

Die Zusammensetzungen gemäß den Beispielen 1 - 6 werden nach üblichen Verfahren, günstig unter Erwärmen auf Temperaturen von etwa 40 - 75° C, verrührt und homogenisiert.

Mit NaOH kann der pH-Wert der Zubereitungen auf Werte im Intervall von etwa 5,0 - 8,0 reguliert werden.

## Patentansprüche

1. Verfahren zur Regulation der Viskosität von Emulsionen, dadurch gekennzeichnet, daß man Emulsionen, welche als Emulgator oder Emulgatoren einen oder mehrere nichtionische Emulgatoren, insbesondere eine oder mehrere Alkancarbonsäure und/oder deren Alkalisalze enthalten, einen oder mehrere Monocarbonsäureester des Di- und/oder Triglycerins bis zum Erreichen der gewünschten Viskosität zusetzt.

2. Verwendung eines oder mehrerer Monocarbonsäureester des Di- und/oder Triglycerins zur Regulation der Viskosität von Emulsionen, welche als Emulgator oder Emulgatoren einen oder mehrere nichtionische Emulgatoren, insbesondere eine oder mehrere Alkancarbonsäuren und/oder deren Alkalisalze enthalten.

3. Verfahren zur Regulation der Viskosität von Emulsionen, dadurch gekennzeichnet, daß man Emulsionen, welche als Emulgator oder Emulgatoren einen oder mehrere Monocarbonsäureester des Di- und/oder Triglycerins enthalten, einen oder mehrere nicht-ionische Emulgatoren, insbesondere eine oder mehrere Alkancarbonsäuren und/oder deren Alkalisalzen bis zum Erreichen der gewünschten Viskosität zusetzt.

4. Verwendung eines oder mehrerer nichtionischer Emulgatoren, insbesondere einer oder mehrerer Alkancarbonsäuren und/oder deren Alkalisalzen zur Regulation der Viskosität von Emulsionen, welche als Emulgator oder Emulgatoren einen oder mehrere Monocarbonsäureester des Di- und/oder Triglycerins enthalten.

## Claims

1. Method of regulating the viscosity of emulsions, characterized in that one or more monocarboxylic esters of di- and/or triglycero are added to emulsions which comprise, as emulsifier or emulsifiers, one or more nonionic emulsifiers, in particular one or more alkanecarboxylic acids and/or alkali metal salts thereof, until the desired viscosity is reached.

2. Use of one or more monocarboxylic esters of di- and/or triglycerol for regulating the viscosity of emulsions which comprise, as emulsifier or emulsifiers, one or more nonionic emulsifiers, in particular one or more alkanecarboxylic acids and/or alkali metal salts thereof.

3. Method of regulating the viscosity of emulsions, characterized in that one or more nonionic emulsifiers, in particular one or more alkanecarboxylic acids and/or alkali metal salts thereof are added to emulsions which comprise, as emulsifier or emulsifiers, one or more monocarboxylic esters of di- and/or triglycerol, until the desired viscosity is reached.

4. Use of one or more nonionic emulsifiers, in particular one or more alkanecarboxylic acids and/or alkali metal salts thereof for regulating the viscosity of emulsions which comprise, as emulsifier or emulsifiers, one or more monocarboxylic esters of di- and/or triglycerol.

## Revendications

1. Procédé de régulation de la viscosité d'émulsions, caractérisé en ce que l'on ajoute un ou plusieurs esters monocarboxyliques du di- et/ou triglycérol à des émulsions qui contiennent, en tant qu'émulsifiant ou émulsifiants, un ou plusieurs émulsifiants non ioniques, en particulier un ou plusieurs acides alcanecarboxyliques et/ou leurs sels alcalins, en vue d'obtenir la viscosité souhaitée.

2. Utilisation d'un ou de plusieurs esters monocarboxyliques du di- et/ou triglycérol en vue de réguler la viscosité d'émulsions qui contiennent, en tant qu'émulsifiant ou émulsifiants, un ou plusieurs émulsifiants non ioniques, en particulier un ou plusieurs acides alcanecarboxyliques et/ou leurs sels alcalins.

3. Procédé de régulation de la viscosité d'émulsions, caractérisé en ce que l'on ajoute un ou plusieurs émulsifiants non ioniques, en particulier un ou plusieurs acides alcanecarboxyliques et/ou leurs sels alcalins à des émulsions qui contiennent, en tant qu'émulsifiant ou émulsifiants, un ou plusieurs esters monocarboxyliques du di- et/ou triglycérol, en vue d'obtenir la viscosité souhaitée.

4. Utilisation d'un ou de plusieurs émulsifiants non ioniques, en particulier d'un ou de plusieurs acides alcanecarboxyliques et/ou de leurs sels alcalins, en vue de réguler la viscosité d'émulsions qui contiennent, en tant qu'émulsifiant ou émulsifiants, un ou plusieurs esters monocarboxyliques du di- et/ou triglycérol.
